# EUROPEAN PATENT SPECIFICATION

(11) **EP 3 115 449 B1**
(45) Date of publication and mention of the grant of the patent: **09.09.2020**
(21) Application number: 16178341.0
(22) Date of filing: 07.07.2016
(51) Int. Cl.: C12M 1/32, B01L 3/00, C12M 3/00

(54) **MULTI-WELL PLATE FOR CELL CULTURES**
MULTIWELL-PLATTER FÜR ZELLKULTUREN
PLAQUETTE À PUITS MULTIPLES POUR CULTURES CELLULAIRES

(30) Priority: 08.07.2015 IT UB20151992
(43) Date of publication of application: 11.01.2017
(73) Proprietor: IVTech S.r.l., 55054 Massarosa (Lucca) (IT)
(72) Inventor: SBRANA, Tommaso, I - 55054 MASSAROSA (Lucca) (IT); GIUSTI, Serena, I - 57034 CAMPO NELL'ELBA (Livorno) (IT); MATTEI, Giorgio, I - 53036 POGGIBONSI (Siena) (IT); CEI, Daniele, I - 56023 NAVACCHIO (Pisa) (IT); AHLUWALIA, Arti, I - 54035 FOSDINOVO (Massa Carrara) (IT)
(74) Representative: Lunati & Mazzoni S.r.L.

(56) References cited:
- CN-U- 202 201 886
- CN-U- 202 482 308
- US-A- 4 948 564
- US-A- 6 096 562

## Description

### MULTI-WELL PLATE FOR CELL CULTURES

The present invention relates to a multi-well plate for cell cultures of the type as recited in the preamble of the first claim.

In detail, the object of the present patent is a plate suitable to be used for analysis of cell cultures in 2D and 3D structures with or without the use of scaffold, ex-vivo tissue explants, slices of tissue or other similar elements, uniquely identified hereinafter by the expression cell cultures.

As is known, multi-well plates consist of a plate on which, in multiple rows, separate wells are made housing cell cultures to be analysed, and a lid is placed over the wells to prevent the contamination thereof.

Multi-well plates are a standard size so as to be easily usable in any reading and investigation system.

They are used primarily for developing in-vitro models of biological tissues/organs, in order to test the action of medicines or active agents on cell cultures and have some significant drawbacks.

Some examples of multi-well plates are described in the patents WO-A-2009/103416, WO-A-2008/143888, US-A-2006/280656, US-A-2007/154357 and US-A-2007/077181. Other examples are US 4948564 A, US 6096562 A and CN 202482308U.

The prior art mentioned above has several significant drawbacks.

A first drawback is that the multi-well plates used today are limited to cultures in static conditions, without the possibility of applying physical and chemical stimuli monitored over time, such as for example varying the degree of oxygenation and/or nutrients inside the well, or applying pressure variations or shear stress.

In addition, the cultures grown in the current multi-well plates are not in communication with each other, which is why it is not possible to study the interaction between different tissues and therefore the possible side effects caused for example by the action of a drug or an active agent. This restricts in particular the accuracy and predictability of data obtained from in-vitro models developed using these supports.

Another important drawback is that any change or the introduction of one or more cell cultures may lead to the contamination of all the cultures present in the multi-well plate.

Indeed, if, for example, a new culture is introduced into a multi-well plate where other cultures are present, the lid must be removed thereby also exposing the other cultures to the risk of contamination.

To avoid such risk, the operator must always take particular care in handling the plate and the lid, avoiding for example passing over the wells already used for other cultures with his hands or other material. In addition, the lid must be positioned with care and particular attention under the laminar flow hood, to avoid possible contamination.

Consequently the use of these systems is highly dependent on the experience and skill of the user.

In this situation the technical purpose of the present invention is to devise a multi-well plate able to substantially overcome the drawbacks mentioned.

Within the sphere of said technical purpose one important aim of the invention is to have a multi-well plate which caters for cultures in dynamic conditions.

An additional purpose of the invention is to devise a plate which makes it possible to place two or more cell cultures grown in different wells in communication with each other.

An additional purpose of the invention is devise a multi-well plate which facilitates the introduction or modification of a cell culture or scaffold without risking contamination of the cultures previously placed in the plate.

The technical purpose and specified aims are achieved by a multi-well plate as claimed in the appended Claim 1.

Preferred embodiments are evident from the dependent claims.

The characteristics and advantages of the invention are clearly evident from the following detailed description of a preferred embodiment thereof, with reference to the accompanying drawings, in which:
**Fig. 1** shows a multil-well plate according to the invention;
**Fig. 2** shows a cross-section of Fig 1.
**Fig. 3** is an exploded view of an assembly of the multi-well plate;
**Fig. 4** shows a cross-section of the assembly of Fig. 1.
**Fig. 5** shows a detail of the multi-well plate according to the invention; and
**Fig. 6** presents a cross-section of a different multi-well plate according to the invention.

Herein, the measures, values, shapes and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape or geometric reference which it is associated with. For example, said terms, if associated with a value, preferably indicate a divergence of not more than 10% of said value.

In addition, where used terms such as "first", "second", "upper", "lower", "main" and "secondary" do not necessarily refer to an order, a priority relationship or relative position, but may simply be used to more clearly distinguish different components from each other.

With reference to said drawings, reference numeral **1** globally denotes the multi-well plate.

It presents a plurality of wells **10** each of which defines a preferred extension axis **10a,** a chamber **10b** suitable to house at least one culture and an introduction aperture **10c** of a cell culture into the well 10.

The wells 10 have extension axes 10a substantially parallel to each other.

They are arranged in one or more rows appropriately substantially parallel to each other and, preferably, practically equidistant from each other.

Preferably, the multi-well plate 1 has twenty-four wells 10 arranged in four rows of six wells 10. It is to be noted how in some cases the multi-well plate 1 may have a different number of rows of wells (appropriately of six wells) optionally at least partly not evenly spaced.

For example, the multi-well plate 1 may have dimensions substantially equal to those of conventional multi-well plates. In particular, it may have a length and a width measured perpendicular to the preferred extension axes 10a, practically respectively comprised between 12 cm and 14 cm and between 7 cm and 10 cm and, more specifically, between 12.5 cm and 13 cm and between 8.3 cm and 8.8 cm.

The multi-well plate 1 comprises a plurality of culture modules **1a** suitable to be constrained, preferably detachably, to each other to form the multi-well plate 1.

In particular, the operator may decide at will, to use a number of modules 1a connected to each other to form a multi-well plate 1 of a length, measured perpendicular to the lying plane of the axes 10a of a row of wells 10, which varies depending on the number of modules 1a used, even equal to the length of a standard multi-well plate. The multi-well plate 1 may thus present different configurations which differ from each other by the number of wells 10 and their size. In particular, such configurations are the same as conventional multi-well plates and described above.

Each culture module 1a comprises a lower body **2** comprising at least one chamber 10b of at least one well 10; an upper body **3** comprising at least one introduction opening 10c of said at least one well 10; and at least one lid **4** suitable to fit into said at least one well 10 leaving only the chamber 10b free. Preferably, each culture module 1a comprises the chambers 10b of the wells 10 arranged in a row; an upper body 3 comprising the introduction openings 10c of the wells 10 arranged in a row; and at least one lid 4 suitable to fit into the wells 10 leaving free only the chamber 10b.

The lower body 2, the upper body 3 and the lid 4 are substantially transparent to light and, in particular, made of glass or other plastic material suitable to permit visibility through them.

The lower body 2 is a single piece of material suitably chosen from polymethylacrylate, polystyrene and polycarbonate (preferably makrolon®).

It defines a longitudinal axis **2a** substantially perpendicular to the preferred extension axes 10a and, in detail, substantially parallel to the lying plane of said axes 10a.

In addition, the lower body 2 further defines an axis **2b** transverse to the longitudinal axis 2a and, preferably, almost perpendicular to the axes, 2a and 10a. The lower body 2 has a length, measured along the preferred extension axis 2a, substantially equal to the width of the multi-well plate 1.

It comprises a base block on which, for each of the wells 10 in a row, a bottom portion **2c** of the well 10 is made including at least the chamber 10b and, appropriately, a supplementary part of the well 10 identifying the engagement portion of said well 10 to the lid 4.

The lower body 2 further comprises at least one channel to place in fluidic through connection two or more chambers 10b of adjacent modules 1a. In particular, it comprises, for each well 10, at least one input channel **21** and at least one output channel **22** suitable to allow a fluid to respectively enter and exit the chamber 10b. Additionally, it may comprise at least one additional channel transverse and, in particular, substantially perpendicular to said channels 21 and 22 to place in fluid through connection two or more chambers 10b of a culture module 1a.

The channels 21 and 22 are substantially parallel to each other and, preferably, have almost the same extension axis. More preferably, the extension axis of the channels 21 and 22 is practically parallel to the transverse axis 2b.

In order to prevent leakages of fluid between adjacent modules 1a and, in particular, during the fluidic passage between the output channel 22 of a culture module 1a and the input channel 21 of the adjacent culture module 1a, the plate 1 comprises hydraulic connectors **5** placed at each output channel 22 and suitable to engage the input channel 21 of an adjacent culture module placing in fluidic connection the channels 21 and 22.

In particular, the connectors 5 are integral with the lower body 2 and protrude from it so as to fit into a bored portion **21a** of the input channel 21 of the adjacent module 2 and detachably constraining it, preferably by interlocking, to said input channel 21.

It should be noted that in the document the term interlocking preferably identifies an interference coupling, i.e. a constraint suitable to ensure interference between the hole, in this case the bored portion 21a, and the element inserted in the hole, in this case the hydraulic connector 5 so as to be airtight and prevent the passage of fluid at the connection area between the hole and element inserted. In detail, the term interlocking identifies a coupling in which the maximum size of the hole is almost less than or, more specifically, substantially equal to the minimum size of the object to be inserted in the hole.

In order to further ensure the creation of a constraint between the connector 5 and the input channel 21, the hydraulic connector 5 may provide for an o-ring or other similar element.

Preferably, the hydraulic connectors 5 are made of a material appropriately chosen from polymethylacrylate, polystyrene and polycarbonate (preferably makrolon®).

Optionally, the multi-well plate 1 comprises one or more hydraulic taps, appropriately three or more ways, suitable to control the fluidic through connection between adjoining chambers 10b and, thus, to adjust the size and control the direction of the flow of liquid between chambers 10b of the plate 1 through the channels 21 and 22.

Said taps are connectable to at least one hydraulic connector 5 so as to place themselves between the channels 21 and 22 of adjacent modules 1a so as to allow the variation at will of the direction of the flow of liquid and thus the topology of the fluidic circuit crossing the chamber 10b and the channels 21 and 22.

It should also be noted that the input 21 and output channels 22 and optionally the hydraulic connectors can place the chambers 10b in fluidic through connection with the outside defining an incoming/outgoing flow from the plate 1 crossing the chambers 10b.

The lower body 2 may also comprise connection means **23** suitable to connect, appropriately along the axes 10a and 2a, two adjacent lower bodies 2.

The connection means 23 may comprise holes made on the lower body 2 extending substantially parallel to the transverse axis 2b; and pins suitable to engage detachably, appropriately by interlocking, with the holes, connecting two adjacent lower bodies 2 to each other.

The upper body 3 comprises, for each of the wells 10 in a row, an apex portion **3a** comprising the introduction section 10c and complementing the bottom portion 2c. It comprises a base plate **31,** appropriately rectangular, defining an extension axis practically parallel to the longitudinal axis 2a; and constraint means **32** suitable to constrain the upper body 3 to a lower body 2 of an adjacent culture module 1a. The plate 31 comprises the apex portions 3a of a row of wells 10.

It defines a support surface of the lower body 2 so as to overlap the apex portions 3a with the bottom portions 2c forming the wells 10.

The plate 31 has a length and width, respectively calculated along the longitudinal 2a and transverse 2b axes, almost identical to the lower body 2.

In particular, the plate 31 and, therefore, the upper body 3 are suitable to rest on the lower body 2 defining, with respect to said lower body 2, a cantilever portion of the upper body 3 suitable to rest on a lower body 2 of an adjacent culture module 1a.

It should be emphasised that the cantilevered portion does not overlap the bottom portions 2c of the adjacent culture module 1a.

The means of constraint 32 are suitable to constrain two adjacent culture modules 1a, appropriately along the transverse axis 2b, placing the longitudinal axes 2a substantially parallel to each other, and preferably the lower bodies 2 substantially in mutual contact.

In particular, they are suitable to constrain the cantilevered portion of the plate 31 engaging the upper body 3 to a lower body 2 of an adjacent culture module 1a. The means of constraint 32 preferably comprise pins suitable to detachably engage, preferably by interlocking, to seats **24** made on the lower body 2.

Said pins may be integral with the plate 31 and, in particular, identify protrusions of the plate 31 appropriately perpendicular to the axes 2a and 2b.

Alternatively, they are separate from the plate 31. In this case the plate 31 presents additional seats on the cantilever portion suitable to overlap the seats 24 so that the pins 32 detachably engage, preferably by interlocking, both seats realising the constraint between the upper body 3 and the lower body 2 of an adjacent culture module 1a.

The upper body 3 comprises, protruding from the plate 31 on the side opposite the support surface of the lower body 2, one or more partitions **33** placed between adjoining introduction sections 10c defining distinct and separate portions of plate each comprising a single section 10c isolated from the other sections 10c; and, appropriately, an at least partial beading **34** of the plate 31 designed to isolate the sections 10c of adjacent upper bodies 3 from each other.

The walls 33 are practically parallel to each other and appropriately extend substantially parallel to the transverse axis 2b.

The beading 34 is preferably made on a portion of the perimeter of the plate 31 and, in particular, on the two sides practically parallel to the transverse axis 2b and on one of the sides substantially parallel to the longitudinal axis 2a.

It has a height, measured along the preferred extension axis, practically at least equal, in detail, practically greater and more specifically, almost at least twice the height, measured along the extension axis 10a, of the partition walls 33.

Lastly, the upper body 3 is in a single piece, preferably in a material suitably chosen from polymethylacrylate, polystyrene and polycarbonate (preferably makrolon®).

The lid 4 is designed to fit into one or more of the wells 10 arranged in a row, through the relevant sections 10c, occupying almost the entire apex portion 3a and partially the bottom portion 2c and therefore, leaving free only the chamber 10b and constraining the upper body 3 to the lower body 2.

It, therefore comprises at least one insert **41** each of which suitable to insert itself, through the introduction section 10c, in a bottom portion 2c detachably constraining itself to it; and at least one head **42** suitable to abut with the upper body 3 and particularly with the plate 31, which are thus enclosed between said head 42 and lower body 2.

Preferably, each culture module 1a comprises a plurality of lids 4, each of which suitable to insert itself, independently of the other lids 4, and to detachably constrain itself to a single well 10 and, consequently, comprising a single insert 41 and a single head 42.

The insert 41 presents a cross-section substantially the same as that of the well 10 so as to detachably constrain itself to it, substantially by interlocking.

The head 42 is suitable to come into direct contact with the plate 31 placing itself between adjacent partition walls 33.

In particular, it has a greater cross-section than the insert 41, and in detail, an almost rectangular shape and, more specifically, almost square with a side appropriately substantially equal to the distance between adjacent partition walls 33.

The insert 41 and the head 42 are in a single piece, preferably in a material appropriately chosen from polymethylacrylate, polystyrene and polycarbonate (preferably makrolon®).

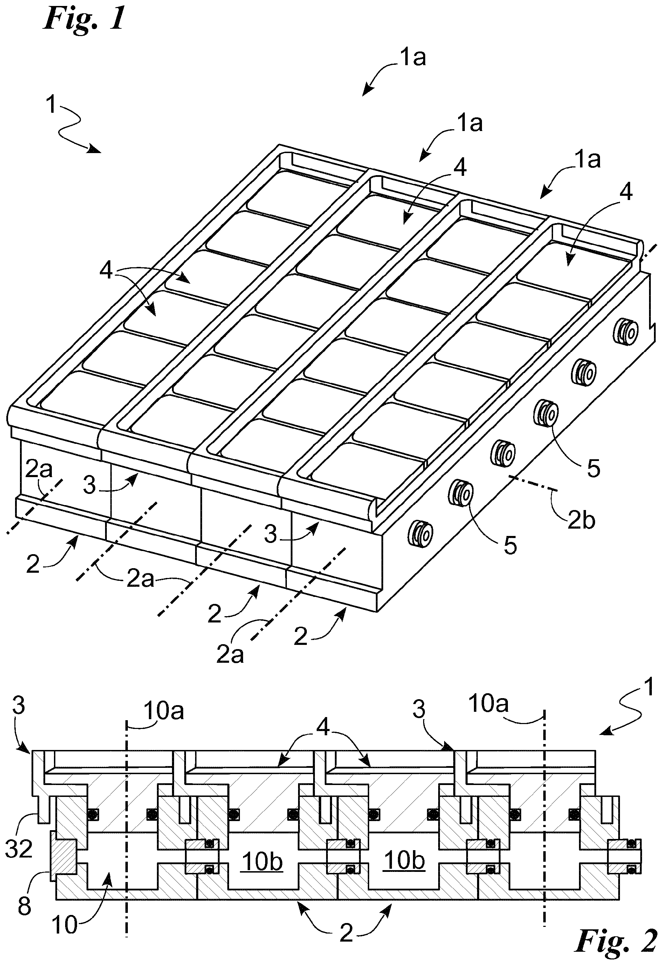

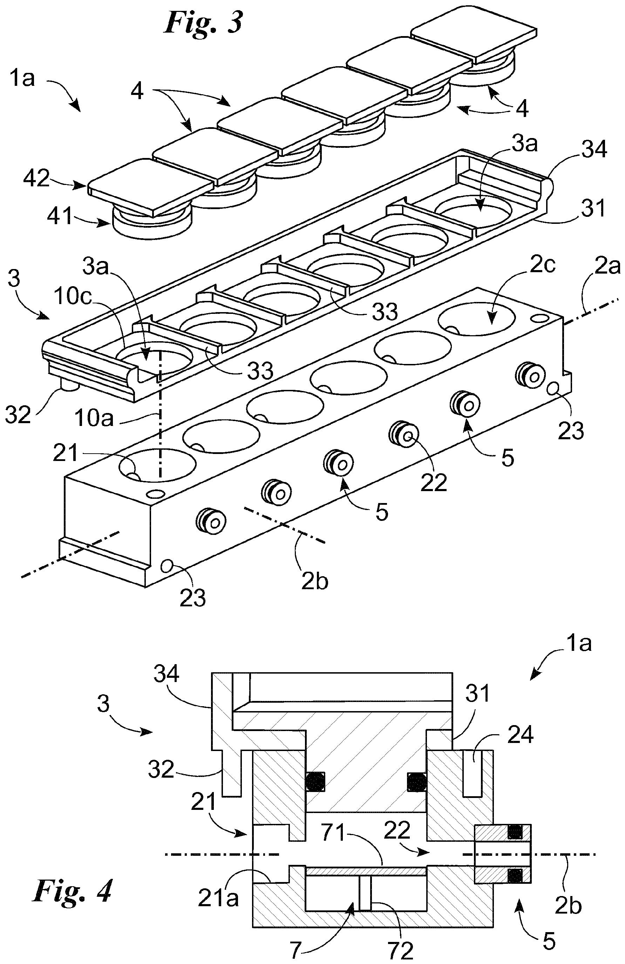

In addition, the lid 4 may comprise a sealing element, such as an o-ring suitable to engage the well 10, increasing the water tightness of the chamber 10b (figs. 2 and 4).

Lastly, the multi-well plate 1 may comprise at least one dividing group **7** suitable to be housed in a chamber 10b so as to divide said chamber 10b into a first sub-chamber, proximal to the input section, and a second sub-chamber proximal to the bottom of the well 10.

The dividing group 7 comprises a membrane **71** suitable to define the partition wall between the sub-chambers; and at least a foot **72** preferably at least three, suitable to rest on the bottom of the well defining the distance between the membrane 71 and the bottom of the well 10, and thus the height of the sub-chambers 10 d and 10e.

The membrane 71 is substantially counter-shaped to the well 10 so that the possible passage of substances and fluids between the sub-chambers is only possible through said membrane.

The membrane 71 is porous, i.e. presents pores suitable to allow the passage between the sub-chambers of substances of a size slightly less than the size of the pores.

It is placed almost parallel to the bottom of the well 10.

The feet 72 have a length substantially between 4 mm and 10 mm.

They are preferably made of a material appropriately chosen from polymethylacrylate, polystyrene and polycarbonate (preferably makrolon®).

It should be noted that, optionally, in order to allow independent feeding from each other, the lower body 2 may provide, for one or more of the chambers 10b, two input channels 21 and two output channels 22 so as to have distinct flows between the sub-chambers.

Finally, to avoid unwanted leakages of fluid, the multi-well plate 1 may provide for caps **8** each of which suitable to detachably engage, preferably by interlocking, to one of the channels 21 and 22 preventing outputs of fluid.

The cap 8 comprises a closure element practically counter-shaped to the channels 21 and 22 so as to detachably engage, by interlocking, to said channels; and, preferably, an abutment suitable to rest on the lower body and, therefore, not fit in the channels 21 and 22 favoring the removal of the caps.

It is made of a material appropriately chosen from polymethylacrylate, polystyrene and polycarbonate (preferably makrolon®).

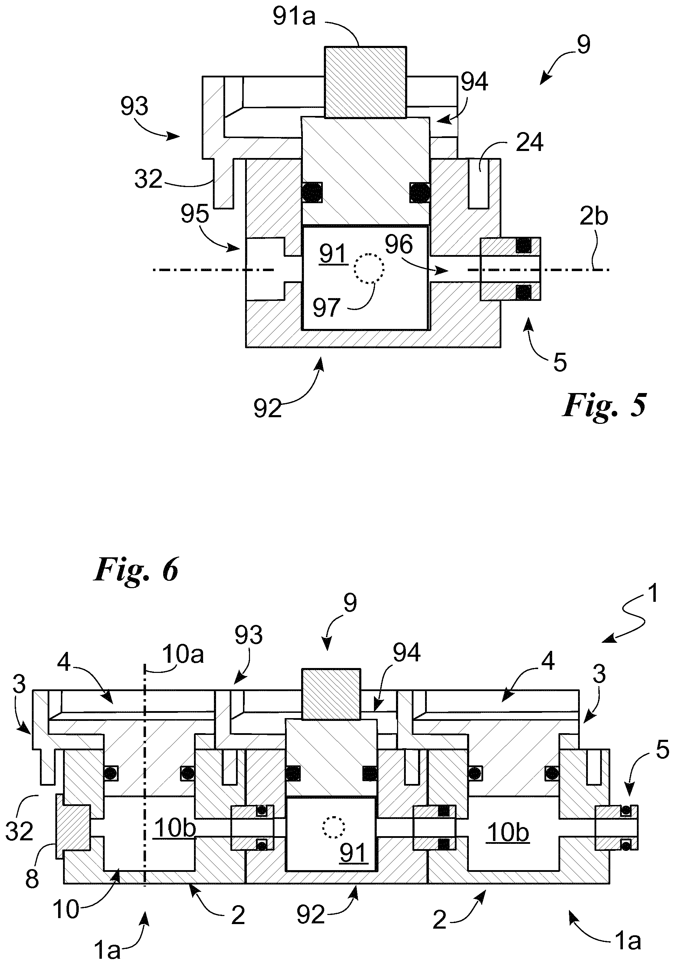

Optionally, the multi-well plate 1 may include at least one adjustment module **9** (Fig. 5) comprising at least one housing and one or more adjustment members 9 and suitable to interpose and constrain itself to two adjacent culture modules 1a placing said at least one housing and said adjustment member 9 in fluidic through connection and interposed to the chambers 10b.

The adjustment module 9 may be structurally analogous to the culture module and comprises an additional lower body **92** defining at least a housing; an additional upper body **93;** and an additional lid **94** designed to fit into said at least one housing constraining the additional lower body 92 to the additional upper body 93. The additional bodies 93 and 92 and the additional lid 94 may be of a material suitably chosen from polymethylacrylate, polystyrene and polycarbonate (preferably makrolon®).

In order to be engageable with one or more adjacent culture modules 1a along the transverse axis 2b, the adjustment module 9 may comprise at least one of the above described constraint means 32 (suitably associated to the upper body 93) and said seats 24 suitably made on the additional lower body 92.

It is to be noted how in the case of an adjustment module 9 connectable to only one culture module 1a, the adjustment module 9 may comprise only one out of the above described constraint means 32 and said seats 24. Alternatively, in the case of an adjustment module 9 which can be constrained between two culture modules 1a, the adjustment module 9 may comprise both the above described constraint means 32 and said seats 24 placed on opposite sides to the means 32 relative to the housing.

The constraint means 32 may comprise one or more pins protruding from cantilevered portions of the additional upper body 93 and suitable to fit into seats 24 made on the lower body 2 of an adjacent culture module 1a.

The additional lower body 92 may include the aforementioned connection means 23 so as to be connected, suitably along the axes 2a and 2b, to adjacent lower bodies 2 and, therefore, to adjacent culture modules 1a.

The additional lid 94 may comprise, in a similar manner to the lid 4, at least one insert, each of which suitable to fit into a housing of the additional lower body 92 detachably engaging itself to it; and, optionally, at least an additional head suitable to abut against the additional upper body 93 and enclosing it between said additional head and the additional lower body 92.

Each adjustment member 91 is suitable to be placed in fluidic through connection between at least one chamber 10b of a first of the culture modules 1a between which the adjustment module 9 and at least one chamber 10b of the second of such culture modules 1a is enclosed, so as to regulate the flow of fluid between the chamber 10b of the first culture module 1a and the chamber 10b of the second culture module 1a.

Each adjustment member 91 is suitable to vary at least the direction (and optionally the orientation) of advancement of the fluid in the multi-well plate 1.

It may comprise a valve, a two or three-way tap, a throttle diaphragm. Preferably, they are three-way valves.

The adjustment member 91 may be provided with control means **91a** suitable to allow an operator to control the adjustment member 91.

For example, the control means 91a may be suitable to control a variation of the direction of fluid flow as a function of a rotation of the adjustment member 91 and, in some cases, of the additional lid 94.

To allow the fluid to reach the housings and, thus, the adjustment members 91 suitable to regulate the flow between the chamber 10b the adjustment module 9 may comprise at least one inlet duct **95** and at least one outlet duct **96** suitable to allow a fluid to enter and exit said seat and, therefore, said adjustment members 91.

The ducts 95 and 96 are substantially parallel to each other and to the channels 21 and 22 and, preferably, have practically the same extension axis. More preferably, the extension axis of the ducts 95 and 96 is practically parallel to the transverse axis 2b.

Optionally, the adjustment module 9 may comprise at least one additional transverse duct **97,** in particular substantially perpendicular to said ducts 95 and 96 and suitable to place in fluid through connection two or more seats and thus two or more adjustment members 91. More optionally, said transverse duct 97 can place in fluidic through connection a seat and, thus, an adjustment member with the outside allowing the insertion and/or extraction of a fluid from the multi-well plate 1.

It should be noted that, in order to avoid leakages of fluid between adjacent modules 1a and, in particular, during the passage of fluid between a channel 21 or 22 and a duct 95 or 96, the multi-well plate 1 may comprise one or more of said hydraulic connectors 5 placed at each outlet duct 22 and suitable to engage to the input channel 21 of a culture module 1a, placing an outlet duct 82 and an input channel 21 in fluidic through connection.

In this case, the connectors 5 are integral with the adjustment module 9 and protrude from it so as to be inserted in said bored portion 21a.

It should also be stressed that the inlet duct 95 can have its own bored engagement portion to the hydraulic connector of a culture module 1a.

The assembly method of a multi-well plate, described above in a structural sense, is as follows.

Initially, the method provides for at least one positioning step in which two or more culture modules 1a are placed alongside along the transverse axis 2b; at least one constraint step of two adjacent culture modules; and at least one anchoring step of the upper body 3 to its lower body 2.

In the positioning step the channels 21 and 22 are placed in connection using the hydraulic connectors 5.

In the constraint step the upper body 3 of a first culture module 1a is placed on a lower body 2 of said first culture module 1a and preferably at the same time, constrained to a second culture module next to the first culture module 1a.

In fact, resting the upper body 3 on a lower body 2 causes the means of constraint 32 to engage the lower body 2 of the second culture module 1a and in particular the pins of the means 32 engage in the seats 24 of the lower body 2 of the second culture module 1a.

In the anchoring step the lid 4 of the first culture module 1a is inserted in one or more of the wells 10 of the first culture module 1a engaging itself to the lower body 2 of the first culture module 1a so as to clamp the upper body 3 of the first culture module 1a between the lower body 2 of the first culture module 1a and said lid 4 of the first culture module 1a.

In particular, at least one insert 41 is inserted through the introduction section 10c, in a bottom portion 2c detachably constraining itself to it and bringing the corresponding head 42 in abutment with the upper body 3 which is thus enclosed and blocked between said head 42 and the lower body 2.

In the case of a multi-well plate 1 with one or more adjustment modules 9 interposed between adjacent culture modules 1a (Fig. 6) the method may provide for a positioning step in which at least one culture module 1a is brought closer along the transverse axis 2b to an adjustment module 9; a constraint step between the culture module 1a and the control module 9.

The functioning of a multi-well plate, described above in a structural sense, is as follows.

Initially, the operator places the upper body 3 on the lower body 2 so as to overlap the portions 2c and 3a forming a row of wells 10.

It should be noted that, the operator, if he wishes to arrange the cultures on a membrane in a chamber, places the dividing group 7 in the chamber 10b, proceeding with the arrangement of the cell culture on the membrane 71.

Alternatively, it is possible to arrange different cultures in the two sub-chambers. In particular, the operator can place a first cell culture on the base of the chamber 10b, position the dividing group 7 in the chamber 10b and place the second cell culture on the membrane 71.

He then places the upper body 3 on the lower body 2 so as to overlap the portions 2c and 3a forming a row of wells 10.

At this point, the operator constrains, for each chamber filled with at least one culture, the lid 4 to the lower body 2 so as to lock the bodies 2 and 3 together, closes the chamber 10b and assembles a culture module 1a. Alternatively, the operator closes each well 10 with a lid 4.

The operator may now proceed with filling the chamber 10b by means of the channels 21 or alternatively fill one or more bottom portions before securing the cover 4.

Once he has prepared the modules 1a, the operator, according to the type of stimulation and the path that he wishes the fluid to take, determines a first order of mutual arrangement of the culture modules 1a. Then, according to said order, he connects, thanks to the hydraulic connectors 5, the channels 21 and 22 of adjacent modules 1a and, thanks to the means of constraint 32, blocks together the culture modules 1a forming the multi-well plate 1.

It should be noted how the operator can use the channels 21 and 22 to place the chambers 10b in connection with an external feeding system so as to control and vary the type, intensity and characteristics of the flow crossing said chambers.

If, for example, the operator wishes to replace one or more of the cell cultures to be fed, he simply disassembles the plate 1, replaces one or more of the previous culture modules 1a with new ones, reassembles the plate using said new culture modules 1a and replenishes the cultures with a different fluid.

It should be noted how the operator, simply by varying the order of the modules 1a and, therefore, without opening the chambers 10b, can change the topology of the circuit thereby varying the model and / or the in-vitro conditions.

The invention achieves important advantages.

A first important advantage is the ability to detach and attach the modules 1a to each other at will, i.e. is to be identified in the highly modular nature and thus the capacity of the multi-well plate 1 to adapt to the needs of the operator.

In fact, the possibility of using only one or more modules 1a, which can be coupled together to make the multi-well plate 1, according to the operator's needs, basically lets you choose the fluidic circuit you want to work with, for example, minimising the volume, and thus the culture medium needed to fill it and minimising the waste of material and money.

Furthermore, the possibility of varying the arrangement of the modules 1a makes it possible to vary, in a simple and fast manner, the order in which a fluid crosses the culture chambers and consequently the tissues placed in communication. Another advantage is the fact that such changes can be made leaving the chamber 10b closed and thus avoiding contamination of the cutures.

An important advantage given by the possibility of varying the modules 1a forming a multi-well plate 1, with or without dividing groups 7 is to be identified in the fact that the operator can vary at will the in-vitro model, retaining the possibility to perform reading and investigation operations by means of automated readers, ensuring the dynamic conditions of the experiment, i.e. the flow of fluid inside the plate 1.

Another advantage related to the modularity of the multi-well plate 1 is to be identified in the fact that if the operator wishes, he can access the chambers 10b individually and, thus expose the cultures grown in the wells 10 to aerosols, according to the experimental needs.

In addition, the operator may provide one or more lids 4 with a hydrodynamic stimulation system or cyclic compression of the cultures and having an immersion body suitable to be immersed in the fluid present in a chamber 10b and a linear actuator suitable to move, preferentially along the axis 10a, the immersion body according to a law of motion as a function of the experimental needs.

A further advantage of no less importance is the modularity of the multi-well plate 1 given by the possibility of varying the culture modules 1a forming a multi-well plate 1 without incurring in contamination of the cell cultures.

Variations may be made to the invention without departing from the scope of the inventive concept described in the independent claims and in the relative technical equivalents. In said sphere all the details may be replaced with equivalent elements and the materials, shapes and dimensions may be as desired.

For example, the lower body 2 may be made in two or more units each of which comprises a single bottom portion 2c and, preferably, the channels 21 and 22. Each unit is lastly fitted with coupling means suitable to allow the units to be mutually detachably constrained, appropriately, by interlocking.

The units are in a material suitably chosen from polymethylmetacrylate, polystyrene and polycarbonate (makrolon®preferably).

## Claims

1. Multi-well plate (1) for cell cultures comprising
- a plurality of wells (10) arranged in rows;
- each of said wells (10) defining a preferred extension axis (10a), a chamber (10b) suitable to house said cell cultures and an introduction section (10c) of said cell cultures in said chamber (10b);
- culture modules (1a) suitable to be fastened together to make said multi-well plate (1); each of said culture modules (1a) comprising a lower body (2) defining a longitudinal axis (2a) substantially perpendicular to said preferred extension axes (10a) and comprising at least a bottom portion (2c) each of which comprising one of said chambers (10a) of said wells (10) arranged along one of said rows;
- **characterised in that** each of said culture modules (1a) comprises
- an upper body (3) suitable to rest on said lower body (2) and comprising a base plate (31) defining an extension axis practically parallel to said longitudinal axis (2a) and comprising a apex portions (3a) and constraint means (32) suitable to constrain said upper body (3) to a lower body (2) of an adjacent culture module (1a); said apex portions (3a) being complementary to said bottom portions (2c) and comprising said introduction sections (10c) of said wells (10) in said row; and
- at least one lid (4) suitable to be inserted, through at least one of said of introduction sections (10c), into at least one of said wells (10b) detachably engaging to said bottom portion (2c) and constraining said upper body (3) to said lower body (2) so as to closing said chamber (10b).

2. Multi-well plate (1) according to claim 1, comprising at least one adjustment module (9) suitable to place itself so as to be between two adjacent culture modules (1a); and wherein said adjustment module comprises at least one adjustment member (91) suitable to be placed in fluidic through connection between at least a chamber(10b) of a first of said culture modules (1a) and at least a chamber (10b) of a second of said culture modules (1a) so as to adjust the flow of fluid between said chamber (10b) of said first culture module (1a) and said chamber (10b) of said second culture module (1a).

3. Multi-well plate (1) according to the preceding claim wherein said at least one adjustment member (91) is suitable to change at least the direction of advancement of said fluid in said multi-well plate (1).

4. Multi-well plate (1) according to the preceding claim, wherein said at least one adjustment member (91) is chosen from a valve, a two and a three-way stopcock.

5. Multi-well plate (1) according to one or more of the preceding claims, wherein said at least one lid (4) is suitable to engage by interlocking to said bottom portion (2c) enclosing said upper body (3) between said lower body (2) and said at least one lid (4).

6. Multi-well plate (1) according to the preceding claim, in which said at least one lid (4) comprises at least one insert (41) each of which suitable to be inserted, through said introduction section (10c) into one of said bottom portions (2c) detachably connecting itself thereto; and at least one head (42) suitable to abut with said upper body (3) which is thus enclosed between said lower body (2) and said head (42).

7. Multi-well plate (1) according to the preceding claim, in which said at least one lid (4) is suitable to engage to a single well (10) and comprises a single insert (41) and a single head (42).

8. Multi-well plate (1) according to one or more of the preceding claims, in which said upper body (3) is suitable to rest on said lower body (2) defining, with respect to said lower body (2), a projecting portion of said upper body (3) adapted to rest on said lower body (2) of an adjacent culture module (1a).

9. Multi-well plate (1) according to one or more of the preceding claims, in which said upper body (3) comprises a base plate (31) comprising said bottom portions (2c); at least one partition wall (33) interposed between adjacent introduction sections (10c) defining distinct and separate portions of said plate (31) each of which comprising a single introduction section (10c).

10. Multi-well plate (1) according to the preceding claim, comprising a rim (34) suitable to isolate introduction sections (10c) of adjacent upper bodies (3) from each other.

11. Multi-well plate (1) according to one or more of the preceding claims, wherein said lower body (2), said upper body (3) and said at least one lid (4) are in a material suitably selected from polymethyl acrylate, polystyrene and polycarbonate.

12. Multi-well plate (1) according to the preceding claim, wherein said lower body (2), said upper body (3) and said at least one lid (4) are transparent to light.

13. Multi-well plate (1) according to one or more of the preceding claims, wherein said lower body (2), for each of said wells (10) in said row, comprises an input channel (21) and an output channel (22) suitable to place in fluidic through connection the chamber (10b) of adjacent culture modules (1a); and wherein said multi-well plate (1) comprises at least one hydraulic tap, suitable to permit control of said fluidic through connection.

14. A method of assembly of a multi-well plate (1) according to one or more of the preceding claims, said assembly method being **characterised in that** it comprises
- at least one positioning step in which at least two of said culture modules (1a) are juxtaposed;
- at least one constraint step wherein said upper body (3) of a first of said culture models (1a) is rested on a lower body (2) of said first culture module (1a) and constrained to the lower body (2) of a second of said culture models (1a) adjacent to said first culture module (1a); and
- at least one anchoring step wherein said upper body (3) of said first culture module (1a) is constrained to said lower body (2) of said first culture module (1a) blocking said upper body (3) of said first culture module (1a) between said upper body (3) of said first culture module (1a) and said lower body (2) of said first culture module (1a).

## Patentansprüche

1. Multiwell-Platter (1) für Zellkulturen, umfassend
- eine Vielzahl an in Reihen angeordneter Wells (10);
- wobei jeder der genannten Wells (10) eine Kammer (10b) definiert, die geeignet ist, die genannten Zellkulturen aufzunehmen, und einen Einsetzabschnitt (10c) zum Einsetzen der genannten Zellkulturen in die genannte Kammer (10b);
- **dadurch gekennzeichnet, dass** er Kulturmodule (1a) umfasst, die geeignet sind, miteinander verbunden zu werden, um den genannten Multiwell-Platter (1) zu bilden;
- **dadurch gekennzeichnet, dass** jedes der genannten Kulturmodule (1a) Folgendes umfasst
- einen unteren Körper (2), umfassend mindestens einen Bodenteil (2c), von denen jeder eine der genannten Kammern (10a) der entlang einer der genannten Reihen angeordneten genannten Wells (10) umfasst;
- einen oberen Körper (3), umfassend zu den genannten Bodenteilen (2c) komplementäre Oberteile (3a), die die genannten Einsetzabschnitte (10c) zum Einsetzen der genannten Wells (10) in die genannte Reihe umfassen, der geeignet ist, auf dem genannten unteren Körper (2) aufzuliegen und geeignet ist, sich lösbar mit einem angrenzenden Kulturmodul (1a) zu verbinden; und mindestens eine Abdeckung (4), die geeignet ist über mindestens einem der genannten Einsetzabschnitte (10c) in mindestens einen der genannten Wells (10) eingesetzt und sich so lösbar mit dem genannten Bodenteil (2c) zu verbinden und so den genannten oberen Körper (3) mit dem genannten unteren Körper (2) zu verbinden.

2. Multiwell-Platter (1) nach Anspruch 1, umfassend mindestens ein Einstellmodul (9), das geeignet ist, zwischen zwei angrenzenden der genannten Kulturmodule (1a) eingesetzt zu werden und zu sein; und bei dem das genannte Einstellmodul mindestens ein Einstellorgan (91) umfasst, das geeignet ist, zur strömungstechnischen Verbindung zwischen mindestens einer Kammer (10b) eines ersten der genannten Kulturmodule (1a) und mindestens einer Kammer (10b) eines zweiten der genannten Kulturmodule (1a) eingesetzt zu werden, um den Flüssigkeitsstrom zwischen der genannten Kammer (10b) des genannten ersten Kulturmoduls (1a) und der genannten Kammer (10b) des genannten zweiten Kulturmoduls (1a) zu regeln.

3. Multiwell-Platter (1) nach dem vorangegangenen Anspruch, bei dem das genannte mindestens eine Einstellorgan (91) geeignet ist, mindestens die Fließrichtung der genannten Flüssigkeit in dem genannten Multiwell-Platter (1) zu ändern

4. Multiwell-Platter (1) nach dem vorangegangenen Anspruch, bei dem das genannte mindestens eine Einstellorgan (91) unter einem Ventil, einem Zwei-Wege-Absperrhahn und einem Drei-Wege-Absperrhahn gewählt wird.

5. Multiwell-Platter (1) nach einem oder mehreren der vorangegangenen Ansprüche, bei dem die genannte mindestens eine Abdeckung (4) geeignet ist sich durch Einrasten mit dem genannten Bodenteil (2c) zu verbinden und so den genannten oberen Körper (3) zwischen dem genannten unteren Körper (2) und der genannten mindestens einen Abdeckung (4) einzuschließen.

6. Multiwell-Platter (1) nach dem vorangegangenen Anspruch, bei dem die genannte mindestens eine Abdeckung (4) mindestens einen Einsatz (41) umfasst, von denen jeder geeignet ist, über den genannten Einsetzabschnitt (10c) in eines der genannten Bodenteile (2c) eingesetzt zu werden und sich mit diesem lösbar zu verbinden; und mindestens ein Kopfteil (42), das geeignet ist, an den genannten oberen Körper (3) anzuschlagen, der so zwischen dem genannten unteren Körper (2) und dem genannten Kopfteil (42) eingeschlossen ist.

7. Multiwell-Platter (1) nach dem vorangegangenen Anspruch, bei dem die genannte mindestens eine Abdeckung (4) geeignet ist, sich mit nur einem Well (10) zu verbinden und nur einen Einsatz (41) und nur ein Kopfteil (42) umfasst.

8. Multiwell-Platter (1) nach einem oder mehreren der vorangegangenen Ansprüche, bei dem der genannte obere Körper (3) geeignet ist, auf dem genannten unteren Körper (2) aufzuliegen und so im Verhältnis zu dem genannten unteren Körper (2) einen vorstehenden Abschnitt des genannten oberen Körpers (3) zu definieren, der geeignet ist, auf dem genannten unteren Körper (2) eines angrenzenden Kulturmoduls (1a) aufzuliegen; und bei dem der genannten obere Körper (3) Verbindungselemente (32) umfasst, die geeignet sind, den genannten vorstehenden Abschnitt des genannten oberen Körpers (3) mit dem genannten unteren Körper (2) des genannten angrenzenden Kulturmoduls (1a) zu verbinden.

9. Multiwell-Platter (1) nach einem oder mehreren der vorangegangenen Ansprüche, bei dem der genannte obere Körper (3) eine die genannten Bodenteile (2c) umfassende Basisplatte (31) umfasst; mindestens eine Trennwand (33) zwischen angrenzenden Einsetzabschnitten (10c), die verschiedene und getrennte Teile der genannten Platte (31) definiert, von denen jeder einen einzigen Einsetzabschnitt (10c) umfasst.

10. Multiwell-Platter (1) nach dem vorangegangenen Anspruch, umfassend eine Umrandung (34), die geeignet ist, die Einsetzabschnitte (10c) der angrenzenden oberen Körper (3) voneinander zu isolieren.

11. Multiwell-Platter (1) nach einem oder mehreren der vorangegangenen Ansprüche, bei dem der genannte untere Körper (2), der genannte oberen Körper (3) und mindestens eine Abdeckung (4) aus einem entsprechend unter Polymethylmethacrylat, Polystyrol und Polycarbonat gewählten Werkstoff bestehen.

12. Multiwell-Platter (1) nach dem vorangegangenen Anspruch, bei dem der genannte untere Körper (2), der genannte obere Körper (3) und die genannte mindestens eine Abdeckung (4) lichtdurchlässig sind.

13. Multiwell-Platter (1) nach einem oder mehreren der vorangegangenen Ansprüche, bei dem der genannte untere Körper (2) für jeden der genannten Wells (10) in der genannten Reihe einen Eingangskanal (21) und einen Ausgangskanal (22) umfasst, die geeignet sind, die Kammer (10b) angrenzender Kulturmodule (1a) strömungstechnisch zu verbinden; und bei dem der genannte Multiwell-Platter (1) mindestens einen hydraulischen Absperrhahn umfasst, der geeignet ist, die genannte strömungstechnische Verbindung zu kontrollieren.

14. Verfahren zur Montage eines Multiwell-Platters (1) nach einem oder mehreren der vorangegangenen Ansprüche; wobei das genannte Verfahren zur Montage **dadurch gekennzeichnet ist,** Folgendes zu umfassen
- mindestens einen Positionierungsschritt, bei dem mindestens zwei der genannten Kulturmodule (1a) nebeneinanderliegen;
- mindestens einen Verbindungsschritt, bei dem der genannte obere Körper (3) eines ersten der genannten Kulturmodule (1a) auf einem unteren Körper (2) des genannten ersten Kulturmoduls (1a) aufliegt und mit dem unteren Körper (2) eines zweiten der genannten Kulturmodule (1a) verbunden ist, das an das genannte erste Kulturmodul (1a) angrenzt; und
- mindestens einen Verankerungsschritt, bei dem der genannte obere Körper (3) des genannten ersten Kulturmoduls (1a) mit dem genannten jeweiligen unteren Körper (2) des genannten ersten Kulturmoduls (1a) verbunden ist und den genannten oberen Körper (3) des genannten ersten Kulturmoduls (1a) zwischen dem genannten oberen Körper (3) des genannten ersten Kulturmoduls (1a) und dem genannten jeweiligen unteren Körper (2) des genannten ersten Kulturmoduls (1a) blockiert.

## Revendications

1. Plaquette à puits multiples (1) pour cultures cellulaires comprenant
- une pluralité de puits (10) disposés en rangées ;
- chacun desdits puits (10) définissant une chambre (10b) apte à contenir lesdites cultures cellulaires et une section d'introduction (10c) desdites cultures cellulaires dans ladite chambre (10b) ;
- **caractérisée en ce qu'**elle comprend des modules de culture (1a) aptes à être liés l'un à l'autre en réalisant ladite plaquette à puits multiples (1) ;
- **en ce que** chacun desdits modules de culture (1a) comprend
- un corps inférieur (2) comprenant au moins une partie de fond (2c) chacune desquelles comprenant une desdites chambres (10a) desdits puits (10) disposés le long d'une desdites rangées ;
- un corps supérieur (3) comprenant des parties de sommet (3a) complémentaires desdites parties de fond (2c) et comprenant lesdites sections d'introduction (10c) desdits puits (10) dans ladite rangée, apte à être en appui sur ledit corps inférieur (2) et apte à se lier de manière amovible à un module de culture (1a) contigu ; et
- au moins un couvercle (4) apte à s'insérer, à travers au moins une desdites sections d'introduction (10c), dans au moins un desdits puits (10) en s'engageant de manière amovible avec ladite partie de fond (2c) et en liant ledit corps supérieur (3) audit corps inférieur (2).

2. Plaquette à puits multiples (1) selon la revendication 1, comprenant au moins un module de réglage (9) apte s'interposer et être entre deux desdits modules de culture contigus (1a) ; et dans laquelle ledit module de réglage comprend au moins un élément de réglage (91) apte à être situé en communication fluide entre au moins une chambre (10b) d'un premier desdits modules de culture (1a) et au moins une chambre (10b) d'un deuxième desdits modules de culture (1a) de façon à régler le débit du fluide entre ladite chambre (10b) dudit premier module de culture (1a) et ladite chambre (10b) dudit deuxième module de culture (1a).

3. Plaquette à puits multiples (1) selon la revendication précédente, dans laquelle ledit au moins un élément de réglage (91) est apte à modifier au moins la direction d'avancement dudit fluide dans ladite plaquette à puits multiples (1).

4. Plaquette à puits multiples (1) selon la revendication précédente, dans laquelle ledit au moins un élément de réglage (91) est choisi parmi une vanne, un robinet à deux voies et un robinet à trois voies.

5. Plaquette à puits multiples (1) selon une ou plusieurs des revendications précédentes, dans laquelle ledit au moins un couvercle (4) est apte à s'engager à emboîtement avec ladite partie de fond (2c) en renfermant ledit corps supérieur (3) entre ledit corps inférieur (2) et ledit au moins un couvercle (4).

6. Plaquette à puits multiples (1) selon la revendication précédente, dans laquelle ledit au moins un couvercle (4) comprend au moins un insert (41) chacun desquels apte à s'insérer, à travers ladite section d'introduction (10c), dans une desdites parties de fond (2c) en se liant de manière amovible avec celle-ci ; et au moins une tête (42) apte à venir en butée contre ledit corps supérieur (3) qui est ainsi renfermé entre lesdits corps inférieur (2) et ladite tête (42).

7. Plaquette à puits multiples (1) selon la revendication précédente, dans laquelle ledit au moins un couvercle (4) est apte à s'engager avec un seul puit (10) et comprend un seul insert (41) et une seule tête (42).

8. Plaquette à puits multiples (1) selon une ou plusieurs des revendications précédentes, dans laquelle ledit corps supérieur (3) est apte à être en appui sur ledit corps inférieur (2) en définissant, par rapport audit corps inférieur (2), une partie en porte-à-faux dudit corps supérieur (3) apte à être en appui sur ledit corps inférieur (2) d'un module de culture contigu (1a) ; et dans laquelle ledit corps supérieur (3) comprend des moyens de raccordement (32) aptes à lier ladite partie en porte-à-faux dudit corps supérieur (3) audit corps inférieur (2) dudit module de culture contigu (1a).

9. Plaquette à puits multiples (1) selon une ou plusieurs des revendications précédentes, dans laquelle ledit corps supérieur (3) comprend une plaquette de base (31) comprenant lesdites parties de fond (2c) ; au moins une paroi de séparation (33) interposées entre les sections d'introduction contiguës (10c) en définissant des parties, distinctes et séparées, de ladite plaquette (31) chacune desquelles comprenant une section d'introduction (10c) unique.

10. Plaquette à puits multiples (1) selon la revendication précédente, une bordure (34) apte à isoler des sections d'introduction (10c) l'une de l'autre des corps supérieurs (3) contigus.

11. Plaquette à puits multiples (1) selon une ou plusieurs des revendications précédentes, dans laquelle ledit corps inférieur (2), ledit corps supérieur (3) et ledit au moins un couvercle (4) sont fait d'un matériau choisi de manière adéquate parmi du polyméthacrylate, du polystyrène et du polycarbonate.

12. Plaquette à puits multiples (1) selon la revendication précédente, dans laquelle ledit corps inférieur (2), ledit corps supérieur (3) et ledit au moins un couvercle (4) sont transparents.

13. Plaquette à puits multiples (1) selon une ou plusieurs des revendications précédentes, dans laquelle ledit corps inférieur (2) comprend, pour chacun desdits puits (10) dans ladite rangée, un conduit d'entrée (21) et un conduit de sortie (22) aptes à relier en communication de fluide la chambre (10b) des modules de culture contigus (1a) ; et dans laquelle ladite plaquette à puits multiples (1) comprend au moins un robinet hydraulique, apte à permettre de contrôler ladite communication fluide.

14. Procédé d'assemblage d'une plaquette à puits multiples (1) selon une ou plusieurs des revendications précédentes ; ledit procédé d'assemblage **étant caractérisé en ce qu'**il comprend
- au moins une étape de positionnement dans laquelle deux desdits modules de culture (1a) sont placés l'un à côté de l'autre ;
- au moins une étape de raccordement dans laquelle ledit corps supérieur (3) d'un premier desdits modules de culture (1a) est en appui sur un corps inférieur (2) dudit premier module de culture (1a) et lié au corps inférieur (2) d'un deuxième desdits modules de culture (1a) contigu audit premier module de culture (1a) ; et
- au moins une étape d'arrimage dans laquelle ledit corps supérieur (3) dudit premier module de culture (1a) est lié audit propre corps inférieur (2) dudit premier module de culture (1a) en bloquant ledit corps supérieur (3) dudit premier module de culture (1a) entre ledit corps supérieur (3) dudit premier module de culture (1a) et ledit propre corps inférieur (2) dudit premier module de culture (1a).
